# EUROPEAN PATENT SPECIFICATION

(11) **EP 1 729 688 B1**
(45) Date of publication and mention of the grant of the patent: **12.08.2015**
(21) Application number: 05729875.4
(22) Date of filing: 24.03.2005
(51) Int. Cl.: A61F 2/24

(54) **CONTROLLED SEPARATION HEART VALVE FRAME**
HERZKLAPPENGERÜST MIT KONTROLLIERTER TEILUNG
STRUCTURE DE VALVULE CARDIAQUE A SEPARATION REGULEE

(30) Priority: 29.03.2004 US 811565
(43) Date of publication of application: 13.12.2006
(73) Proprietor: Edwards Lifesciences Corporation, Irvine, CA 92614 (US)
(72) Inventor: MARQUEZ, Salvador, Foothill Ranch, CA 92610 (US)
(74) Representative: Potter Clarkson LLP
(86) International application number: PCT/US2005/009863
(87) International publication number: WO 2005/097003

(56) References cited:
- US-A- 3 714 671
- US-A1- 2003 014 105
- US-B1- 6 635 085
- US-B2- 6 558 418

## Description

### Field of the Invention

The present invention pertains to an internal support frame for a prosthetic heart valve and, more particularly, to a leaflet support frame that separates into cusps after implantation.

### Background of the Invention

Prosthetic heart valves are used to replace damaged or diseased heart valves. In vertebrate animals, the heart is a hollow muscular organ having four pumping chambers: the left and right atria and the left and right ventricles, each provided with its own one-way outflow valve. The natural heart valves are identified as the aortic, mitral (or bicuspid), tricuspid and pulmonary valves. The valves of the heart separate chambers therein, and are each mounted in an annulus therebetween. The annuluses comprise dense fibrous rings attached either directly or indirectly to the atrial and ventricular muscle fibers. In a valve replacement operation, the damaged leaflets are typically excised and the annulus sculpted to receive a replacement valve.

The four valves separate each ventricle from its associated atrium, or from the ascending aorta (left ventricle) or pulmonary artery (right ventricle). After the leaflets have been excised, the annulus generally comprises a ledge extending into and defining the orifice between the respective chambers. Prosthetic valves may attach on the upstream or downstream sides of the annulus ledge, but preferably reside outside of the ventricles to avoid interfering with the large contractions therein. Thus, for example, in the left ventricle a prosthetic valve is preferably positioned on the inflow side of the mitral valve annulus (toward the left atrium), or on the outflow side of the aortic valve annulus (toward the ascending aorta).

One of the primary types of heart valve prostheses is a tissue-type or "bioprosthetic" valve which is constructed with natural-tissue valve leaflets (whole valve, e.g., porcine, or a plurality of leaflets, e.g., from bovine equine or other pericardium) which function much like a natural human heart valve, imitating the natural action of the flexible heart valve leaflets which seal against each other to ensure the one-way blood flow. Synthetic leaflets have also been proposed, and thus the term "flexible leaflet valve" refers to both natural and artificial "tissue-type" valves. Two or more flexible leaflets are mounted within a peripheral support structure that usually includes posts or commissures extending in the outflow direction to mimic natural fibrous commissures in the native annulus. Components of the valve are usually assembled with one or more biocompatible fabric (e.g., Dacron) coverings, and a fabric-covered sewing ring is typically provided on the inflow end of the peripheral support structure.

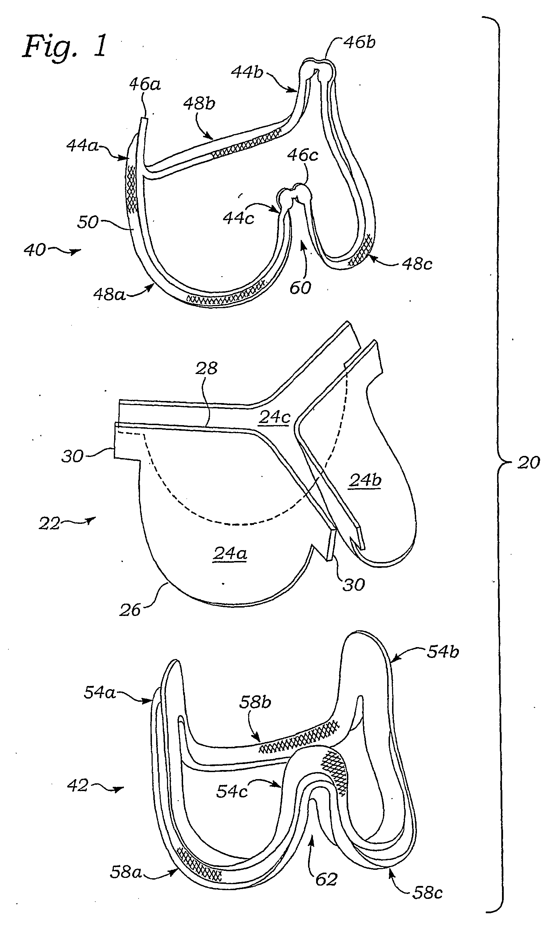

In most bioprosthetic-type valves, a metallic or polymeric structure provides base support for the flexible leaflets, which extend therefrom. One such support is an elastic "support frame," sometimes called a "wireform" or "stent," which has a plurality (typically three) of large radius cusps supporting the cusp regions of each flexible leaflet. The ends of each pair of adjacent cusps converge somewhat asymptotically to form upstanding commissures that terminate in tips, each extending in the opposite direction as the arcuate cusps and having a relatively smaller radius. The support frame typically describes a conical tube with the commissure tips at the small diameter end. This provides an undulating reference shape around which a fixed edge of each leaflet attaches (via components such as fabric and sutures) much like the natural fibrous skeleton in the aortic annulus. One example of the construction of a flexible leaflet valve is seen in U.S. Patent No. 5,928,281 to Huynh, et al. (Edwards Lifesciences, Corp., Irvine, CA), in which the exploded view of Fig. 1 illustrates a fabric-covered wireform 54 and a fabric-covered support stent 56 on either side of a leaflet subassembly 52.

US 6,635,085 relates to wire stents for reinforcing flexible leaflet prosthetic heart valves. In particular this document teaches a stent member which has a plurality of leg members extending from the stent member. The legs help stabilize and locate the stent during the moulding process. After this process, the legs can be removed from the stent.

US 3,714,671 relates to a specially structured heart valve which comprises a tricuspid valve and a graft support ring or stent therefore.

US 2003/014105 relates to a flexible prosthetic tissue-type heart valve having commissures that are substantially decoupled from a cusp support structure.

Because of the rigidity of the material used in the support frame, conventional valves have a diameter that is minimally affected by the natural motion of the heart orifice. In the aortic position, the commissures extend in the downstream direction a spaced distance from the walls of the downstream aortic wall. Movement of the aortic wall or sinuses does not substantially affect movement of the cantilevered commissures, though fluid flow and pressures generated by movement of the walls ultimately does cause the commissures to dynamically flex to some extent (i.e., they are cantilevered downstream in the aorta). Natural dilatation of the annulus is therefore restricted, imposing an artificial narrowing of the orifice, and increasing the pressure drop therethrough.

Some flexible leaflet prosthetic heart valves are designed to be relatively more flexible. For example, U.S. Patent No. 4,106,129 to Carpentier, et al. discloses a heart valve with a compliant supporting stent capable of annular deformation. The stent is made with a single flexible wire pre-formed to define inverted U-shaped commissures supports merging smoothly with connecting arcuate portions. The heart valve in Carpentier, et al. is capable of yielding to a limited extent in response to forces which tend to alter the configuration and circumference of the supporting stent. Figs. 9 and 10 of the '129 Patent illustrate aortic and mitral embodiments, respectively, with sewing rings adapted to attach to the particular annulus.

More recently, U.S. patent No. 6,558,418 to Carpentier, et al. discloses a highly flexible tissue-type heart valve having a structural stent in a generally cylindrical configuration with cusps and commissures that are permitted to move radially. The stent commissures are constructed so that the cusps are pivotably or flexibly coupled together at the commissures to permit relative movement therebetween. The prosthetic valve of the '418 patent is designed such that alternating peripheral portions are attached to the aortic annular region and the sinus region, and the flexible valve accommodates the in-and-out movements of both regions. The structural stent is useful during implantation to maintain the valve shape for proper suturing, and to provide a barrier to leaflet placations. However, once implanted, the structural stent still provides an impediment to complete flexibility.

Accordingly, there is a need for a highly flexible heart valve that responds to and does not significantly impede the natural motions of the annulus and adjacent vessel walls but which also maintains a desired shape during implantation

### Summary of the Invention

The present invention provides an improved leaflet support frame for a prosthetic heart valve that maintains its shape during implantation but eventually separates into a plurality of cusps thereafter. The support frame includes alternating cusps and commissures with a plurality of flexible leaflets secured along their cusp edges to the frame, one per frame cusp. The frame commissures are designed to fracture at a period after implantation such that the cusps move substantially independently of each other. In contrast to earlier biodegradable cusp connections, the frame commissures have substantially the same material stiffness in bending as the frame cusps so as to substantially limit the possibility of the valve cusps prematurely breaking free of each other from the inevitable manipulation of the valve during implantation.

In accordance with the present invention, a support frame for a flexible leaflet prosthetic heart valve is provided wherein the support frame is a single, continuous, element and that has a plurality of cusps each sized and shaped to support a cusp of a flexible leaflet of the heart valve, and a plurality of commissures, one between each adjacent pair of cusps. The commissures and cusps have substantially the same material stiffness in bending, but the commissures each have a point of weakness designed to fracture upon repeated relative movement of the cusps after implantation thereby permitting the cusps to move substantially independently of each other.

Preferably, the support frame is made of a flexible material such as Nitinol.

Preferably, each cusp of the support frame transitions into two commissure regions, and wherein the point of weakness at the commissures comprises a frangible bridge between adjacent commissure regions. The frangible bridge may comprise a narrow portion of the support frame relative to adjacent portions, or may be a notch. Desirably, the commissure regions terminate in enlarged ears on either side of the frangible bridge. The support frame may be covered with a biocompatible fabric, and the enlarged ears are sized to prevent the commissure regions from poking through the fabric once the frangible bridge has fractured.

Also described herein is a method of replacement of a natural heart valve with a flexible leaflet prosthetic heart valve. The method includes providing a flexible leaflet prosthetic heart valve having an internal support frame with alternating cusps and commissures, the cusps of the flexible leaflets being attached along the support frame cusps. The commissures of the internal support frame are designed to fracture upon repeated relative movement of the cusps after implantation such that the support frame cusps can move substantially independently of each other. The method includes implanting the flexible leaflet prosthetic heart valve.

Desirably, the internal support frame is made of a continuous flexible element which will withstand and spring back from substantial compressive forces imparted thereon during implantation. The step of implanting the flexible leaflet prosthetic heart valve may comprise compressing the valve and delivering it to the site of implantation through a tube in a less-invasive procedure. In one version, the flexible leaflet prosthetic heart valve is designed to be implanted in the aortic position and further includes a sewing band that follows the alternating cusps and commissures of the support frame. The step of implanting the heart valve therefore comprises attaching the sewing band up and down the fibrous cusps and commissures of the natural aortic annulus and ascending aorta. In accordance with an exemplary embodiment, the commissures of the internal support frame are designed to fracture from between approximately two days and two weeks after implantation.

### Brief Description of the Drawings

Fig. 1 is an exploded view of components of a prosthetic heart valve of the present invention;
Fig. 2 is a perspective view of an exemplary leaflet support frame of the present invention seen from an outflow end;
Fig. 2A is an enlarged view of one of the commissure tips of the leaflet support frame of Fig. 2; and
Fig. 3 is a perspective view of the leaflet support frame seen from an inflow end.

### Description of the Preferred Embodiments

According to the present invention there is provided a support frame according to claim to 1.

The present invention provides a highly flexible prosthetic heart valve that is primarily suited for implantation in the aortic position. In particular, the prosthetic heart valve has an attachment or sewing band that follows the undulating shape of an internal support frame. As such, the sewing band may be attached (e.g., with sutures) up and down the fibrous cusps and commissures of the natural aortic annulus and ascending acrta. It should be understood, however, that the characteristics of the heart valve of the present invention may also be suitable for use in other valve locations, such as in the tricuspid position.

The prosthetic heart valve is highly flexible and includes an internal frame that supports a plurality of flexible leaflets providing occluding surfaces for the valve. The internal frame is designed to break apart into separate cusps, one per leaflet, after implantation to render the valve even more flexible. One example of construction of a prosthetic heart valve is shown and described below, although it should be understood that the invention is not limited by a particular valve construction. Any valve that has flexible leaflets supported around their periphery may include an internal frame that separates into parts after implantation. A particular preferred construction of a flexible heart valve, aside from substitution of the separable internal support frame described herein, is disclosed in U.S. Patent No. 6,558,418 to Carpentier, et al.

As mentioned above, the term "flexible leaflet" refers to any valve leaflet that flexes and "coapts" against the other leaflets to close the valve, much like the native leaflets. Because of the separable internal support frame and relative cusp movements, at least the coapting edges of the leaflets must be somewhat flexible to accommodate the motion of the peripheral support structure. Preferably, the flexible leaflets are separate synthetic (e.g., polymer) or natural (e.g., pericardium) leaflets attached to the peripheral support structure. However, whole natural valves such as xenograft porcine valves may be used. Also, it is conceivable that a synthetic leaflet that is partly rigid but flexible along its coapting edge may be used.

Fig. 1 illustrates in exploded view primary components of an exemplary prosthetic heart valve 20 of the present invention. The heart valve 20 includes a subassembly of flexible leaflets 22, preferably comprising three separate leaflets 24a, 24b, 24c. Each leaflet includes an arcuate cusp edge 26 on an inflow end opposite a free or coapting edge 28 on an outflow end. In the exemplary embodiment, a pair of tabs 30 extend outward from either end of the coapting edge 28. As explained in U.S. Patent No. 6,558,418, such tabs can be used to secure the leaflets 24 to commissures of a peripheral support frame in a manner that reduces stress in the attachment sutures. Each tab on the leaflets is juxtaposed against a tab on an adjacent leaflet so that there are three pairs of contacting tabs extending outward approximately 120 degrees apart in the leaflet subassembly 22.

The leaflet subassembly 22 (or separate leaflets 24) is held in place within the valve 20 by a support structure comprising a cloth-covered support frame 40 and a sewing band 42. The sewing band 42 is also desirably covered with cloth or some other material that aids connectivity and/or biocompatibility. The support frame 40 and sewing band 42 are similarly shaped with a plurality, preferably three, posts or commissures extending in an outflow direction and a plurality of arcuate cusps on an inflow end. As can be seen from Fig. 1, the arcuate cusps of the support structure correspond to be leaflet cusps 26.

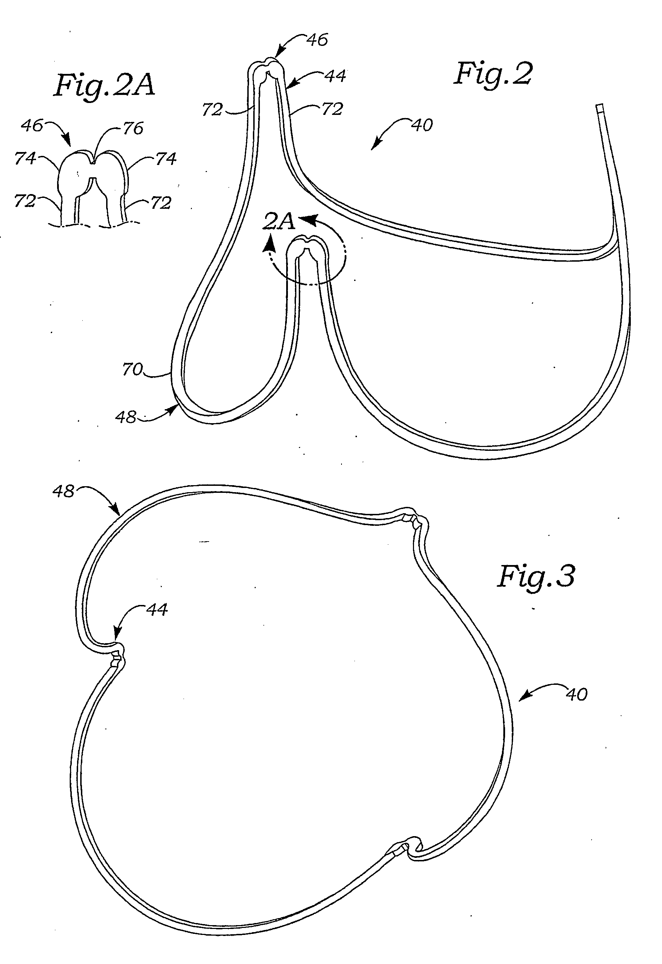

The exemplary leaflet support frame 40 (shown without its cloth cover in Figs. 2 and 3) includes three upstanding commissures 44a, 44b, 44c terminating in tips 46a, 46b, 46c, and three arcuate cusps 48a, 48b, 48c. Although not clearly shown, the surface of revolution defined by the leaflet support frame 40 is desirably a cylinder or more preferably, a cone, with the commissure tips 46 being disposed slightly radially closer together than the apices of the arcuate cusps 48. A cloth cover seen in Fig. 1 closely surrounds the support frame 40 and preferably provides an outwardly extending flap 50 that is used to secure the valve components together. The material of the cloth cover may be any biocompatible fabric, preferably a polymer fabric such as polyethylene terepthalate (PET). Greater details of the leaflet support frame 40, a primary subject of this invention, are provided below.

The sewing band 42 also has three upstanding commissures 54a, 54b, 54c terminating in tips 56a, 56b, 56c, and three arcuate cusps 58a, 58b, 58c. A variety of suture-permeable materials may be used, although a molded silicone core covered with a fabric (e.g., PET) is preferred.

When assembled, the leaflets 24 are sandwiched between and attached to both the support frame 40 and sewing band 42. Sutures are typically used to join the outwardly projecting flap 50 of the support frame 40 through the cusp edges 26 of the leaflets 24 and through the cloth-covered sewing band 42. Although not shown, each pair of leaflet tabs 30 projects outward through gaps 60 in the support frame 40 and may be folded away from one another so as to lie to the inside of each of the commissures 54 of the sewing band 42 in the assembled valve 20. Again, although various construction details may be modified, each leaflet 24 is desirably generally continuously attached around the support frame 40 along the cusp edge 26 and at the two tabs 30. The coapting edge 28 remains free.

Now with reference to Figs. 2 and 3, the support frame 40 is fabricated as a single, continuous, integral, wire-like element 70 of a homogeneous material. One particularly desirable material is Nitinol, and a preferred fabrication technique is to laser-cut a 2-dimensional blank from a sheet, or a 3-dimensional blank from a tube, and then bend and heat treat the blank into the illustrated shape. Further details on this technique can be seen in U.S. patent application No. 10/423,019, filed April 24, 2003. Using such techniques, the cross-section of the element 70 will typically be square or rectilinear, although electropolishing is desirably performed to microscopically round the corners.

Nitinol is preferred because of its biocompatibility combined with flexible qualities. That is, the support frame 40 must be relatively stiff to maintain the valve shape during implantation when the surgeon is attempting to manipulate and attach the valve into place. At the same time, the support frame 40 should be capable of springing back from the sometimes strong forces experienced during the implantation procedure. Alternatively, the support frame 40 may be made of a single, continuous piece of an alloy of carbon, silicon, phosphorus, sulphur, chromium, nickel, beryllium, cobalt, iron, manganese and molybdenum which is sold under the ELGILOY trade name by Elgiloy, L.P. of Elgin, Ill., U.S.A. Another potentially useful material is titanium or an alloy thereof. The support frame 40 could also be molded from a polymer, such as DELRIN, or any other biocompatible material exhibiting appropriate flexibility.

Fig. 2A is an enlargement of one of the commissure tips 46. In a preferred embodiment, the cross-section of the wire-like element 70 is substantially constant around the entire support frame 40 except at the tips 46. Each arcuate cusp 48a, 48b, 48c transitions into a commissure region 72 adjacent to the tips 46. The commissure regions are generally linear and extend upward to an enlarged ear 74. Adjacent ears 74 join across a relatively weak point or frangible bridge 76 at the center line of the commissure tip 46. The frangible bridge 76 comprises a narrowing of the cross-section of the elongated element 70 relative to the rest of the element The bridge 76 is designed to fatigue and fracture after the valve is implanted so that the cusps 48 separate, or in other words, become free to move substantially independently of each other (though they will preferably remain coupled via the sewing band 42 or surrounding fabric).

For a support frame 40 fabricated as a continuous Nitinol element, the bridge 76 desirably has a cross-section of approximately 0.381 mm (0.015") in height (axial dimension) and 0.508 mm (0.020") thickness (radial dimension). Although the length of the bridge 76 does impact the moment arm from the ears 74, and thus any stress applied thereto after implantation the length is relatively small (approximately the same as the height) and small changes will not greatly alter the stress. With this cross-section, the bridge 76 is designed to fatigue and fracture anywhere between about 2 days and 2 weeks after implantation (or equivalent number of cycles at a heart beat rate 1 Hz). Those of skill in the art will understand that depending on the material/construction and desired time to fracture, the specific dimensions of the bridge 76 may vary. In the illustrated embodiment, the frangible bridge 76 comprises a narrow length of material between the ears 72, but another technique is to provide a notch or other such feature that creates a stress point in the bridge.

An alternative construction process is to form three separate cusps and to connect them at the frangible bridges 76. To provide the benefits of the invention the connection should have a stiffness/rigidity similar to a bridge formed integrally with the cusps 48. That is, the commissure tips 46a, 46b, 46c should be relatively rigid at implant to prevent the arcuate cusps 48a, 48b, 48c from unduly pivoting with respect to one another. Of course, some cusp pivoting may occur due to the inherent flexibility of the support frame 40, just as long as the commissure tips 46a, 46b, 46c unduly do not increase that flexibility. For example, a welded connection between adjacent ears 74 might be designed to break some time after implantation. Alternatively, a separate member forming the bridge 76 might be connected at each end to the surrounding ears 74 by welding or threading, for example. It should be clear that there are various ways to fabricate a support frame 40 that has commissures and cusps with substantially the same material stiffness in bending but a point of weakness at each commissure designed to fracture upon repeated relative movement of the cusps after implantation such that the cusps separate.

In another aspect of the invention, the enlarged ears 74 help prevent the separated cusps 48 of the support frame 40 from poking through the surrounding fabric. In a preferred embodiment, the enlarged ears 74 desirably have a radius of about 0.5-1.5 mm, preferably about 1.0 mm, to prevent fabric poke-through.

The present heart valve is used in a surgical method of replacing a natural heart valve. Any of the aforementioned heart valves are implanted in the appropriate location (e.g., in the aortic annulus and ascending aorta) using traditional open-chest surgery, or a less-invasive surgery such as a mini-thoracotomy, or even percutaneously. If the support frame 40 of the heart valve is made of a highly flexible material such as Nitinol, the valve may be compressed to a relatively small package and inserted using a minimally-invasive technique, such as percutaneously through a catheter passed up through the femoral artery. Another technique that may be used is through a port access incision in the chest. One particularly advantageous aspect of the invention is that the support frame allows for the bending of the frame at the commissure tips during implantation, but because it is a continuous or at least unitary piece, does not allow for relative axial motion of the commissures.

Once in position, the surgeon secures the valve to the surrounding anatomy using sutures, staples, or other such attachment structures as are known in the field. Depending on the technique used, the attachment structure may be manipulated manually, or via robotic assistance.

It will also be appreciated by those of skill in the relevant art that various modifications or changes may be made to the examples and embodiments described without departing from the intended scope of the invention.

## Claims

1. A support frame (10) for a flexible leaf et prosthetic heart valve;
a plurality of cusps (48) each sized and shaped to support a cusp of a flexible leaflet of the heart valve; and
a plurality of commissures (44) one each between each adjacent pair of cusps (48), **characterised by** commissures (44) each having a point of weakness designed to fracture upon repeated relative movement of the cusps after implantation such that the cusps (48) move substantially independently of each other and wherein the support frame is a single, continuous, element comprising:

2. The support frame (40) of claim 1, wherein the support frame (40) is formed from a continuous, homogeneous material.

3. The support frame (40) of claim 2, wherein the commissures (44) and cusps (48) have substantially the same material stiffness in bending prior to reaching the point of fatigue.

4. The support frame (40) of claim 1, wherein the support frame (40) is made of Nitinol.

5. The support frame (40) of claim 1, wherein each cusp (48) of the support frame (40) transitions into two commissure regions, and wherein the point of weakness at the commissures (44) comprises a frangible bridge (76) between adjacent commissure regions.

6. The support frame (40) of claim 5, wherein the frangible bridge (76) comprises a narrow portion of the support frame (4C) relative to adjacent portions.

7. The support frame (40) of claim 5, wherein the point of weakness comprises a notch.

8. The support frame (40) of claim 5, wherein the commissure regions terminate in enlarged ears (74) on either side of the frangible bridge (76).

9. The support frame (40) of claim 8, further including a biocompatible fabric covering the support frame (40) and wherein the enlarged ears (74) are sized to prevent the commissure regions from poking through the fabric once the frangible bridge (76) has fractured.

## Patentansprüche

1. Stützrahmen (40) für eine Herzklappenprothese mit flexiblem Flügel, umfassend:
mehrere Klappensegel (48), die jeweils dafür bemessen und geformt sind, ein Klappensegel eines flexiblen Flügels der Herzklappe zu stützen, und
mehrere Kommissuren (44), jeweils eine zwischen jedem benachbarten Paar von Klappensegeln (48), **dadurch gekennzeichnet, dass** die Kommissuren (44) jeweils einen Schwachpunkt haben, der dafür gestaltet ist, bei wiederholter Relativbewegung der Klappensegel nach der Implantierung zu brechen, sodass die Klappensegel (48) sich im Wesentlichen unabhängig voneinander bewegen, und wobei der Stützrahmen ein einzelnes, durchgängiges Element ist.

2. Stützrahmen (40) nach Anspruch 1, wobei der Stützrahmen (40) aus einem durchgehenden, homogenen Material gebildet ist.

3. Stützrahmen (40) nach Anspruch 2, wobei die Kommissuren (44) und Klappensegel (48) im Wesentlichen die gleiche Materialsteifigkeit beim Biegen vor dem Erreichen des Ermüdungspunkt aufweisen.

4. Stützrahmen (40) nach Anspruch 1, wobei der Stützrahmen (40) aus Nitinol besteht.

5. Stützrahmen (40) nach Anspruch 1, wobei jedes Klappensegel (48) des Stützrahmens (40) in zwei Kommissurbereiche übergeht und wobei der Schwachpunkt an den Kommissuren (44) eine zerbrechliche Brücke (76) zwischen benachbarten Kommissurbereichen umfasst.

6. Stützrahmen (40) nach Anspruch 5, wobei die zerbrechliche Brücke (76) einen im Verhältnis zu benachbarten Abschnitten schmalen Abschnitt des Stützrahmens (40) umfasst.

7. Stützrahmen (40) nach Anspruch 5, wobei der Schwachpunkt eine Kerbe umfasst.

8. Stützrahmen (40) nach Anspruch 5, wobei die Kommissurbereiche in vergrößerten Ohren (74) auf beiden Seiten der zerbrechlichen Brücke (76) enden.

9. Stützrahmen (40) nach Anspruch 8, ferner ein biokompatibles Flächengebilde umfassend, das den Stützrahmen (40) bedeckt und wobei die vergrößerten Ohren (74) dafür bemessen sind, zu verhindern, dass die Kommissurbereiche durch das Flächengebilde stoßen, sobald die zerbrechliche Brücke (76) gebrochen ist.

## Revendications

1. Structure de support (40) pour une valvule cardiaque prothétique à valve flexible comprenant:
une pluralité de lames (48) chacune dimensionnée et formée pour supporter une lame d'une valve flexible de la valvule cardiaque ; et
une pluralité de commissures (44), une entre chaque paire adjacente de lames (48), **caractérisée par** des commissures (44) ayant chacune un point plus fragile conçu pour se rompre lors d'un mouvement relatif répété des lames après implantation de sorte que les lames (48) se déplacent sensiblement indépendamment les unes des autres et dans laquelle la structure de support est un élément continu unique.

2. Structure de support (40) selon la revendication 1, dans laquelle la structure de support (40) est constituée d'un matériau homogène continu.

3. Structure de support (40) selon la revendication 2, dans laquelle les commissures (44) et les lames (48) ont sensiblement la même rigidité en flexion de matériau avant d'atteindre le point de fatigue.

4. Structure de support (40) selon la revendication 1, dans laquelle la structure de support (40) est constituée de Nitinol.

5. Structure de support (40) selon la revendication 1, dans laquelle chaque lame (48) de la structure de support (40) se prolonge par deux régions de commissure, et dans laquelle le point plus fragile au niveau des commissures (44) comprend un pont cassant (76) entre des régions de commissure adjacentes.

6. Structure de support (40) selon la revendication 5, dans laquelle le pont cassant (76) comprend une partie étroite de la structure de support (40) par rapport à des parties adjacentes.

7. Structure de support (40) selon la revendication 5, dans laquelle le point plus fragile comprend une encoche.

8. Structure de support (40) selon la revendication 5, dans laquelle les régions de commissure se terminent dans des pattes agrandies (74) de chaque côté du point cassant (76).

9. Structure de support (40) selon la revendication 8, comprenant en outre une étoffe biocompatible couvrant la structure de support (40) et dans laquelle les pattes agrandies (74) sont dimensionnées pour empêcher les régions de commissure de passer à travers l'étoffe une fois que le pont cassant (76) s'est cassé.
